# EUROPEAN PATENT APPLICATION

(11) **EP 2 932 963 A1**
(43) Date of publication of application: **21.10.2015**
(21) Application number: 14001400.2
(22) Date of filing: 16.04.2014
(51) Int. Cl.: A61K 9/20, A61K 47/38, A61K 31/198

(54) **Stable pharmaceutical dosage forms comprising Levothyroxine sodium**

(71) Applicant: Uni-Pharma Kleon Tsetis Pharmaceutical Laboratories S.A., 145 64 Kifissia (GR)
(72) Inventor: Tseti, Ioulia, 145 61 Kifissia (GR)
(74) Representative: Wibbelmann, Jobst

(57) **Abstract**

The present invention relates to a novel and stable levothyroxine sodium pharmaceutical composition and belongs to the technical field of pharmaceutical preparation and in particular to a levothyroxine sodium tablets. In the current invention, the levothyroxine sodium dosage forms comprise as active ingredient levothyroxine sodium in an amount of about 0.01% w/w to about 1% w/w and as excipients Microcrystalline cellulose in an amount of 80% w/w to 95% w/w , disintegrant in an amount of 5% w/w to 10% w/w , glidant in an amount of 0.5% w/w to 3% w/w , and lubricant in an amount of 0.5% w/w to 2% w/w .

The proposed composition by improving the stability and the dissolution of the preparation can benefit the clinical application.

Finally, the present invention is directed to therapeutic agents for treatment of hormone disorders and relates to stable pharmaceutical dosage forms of Levothyroxine sodium.

## Description

### Background of the Invention

Levothyroxine Sodium is a hormone produced by the thyroid gland and is used to treat and control the course of various diseases of the thyroid. The chemical name of levothyroxine sodium is (S)-2-amino-3-[4-(4-hydroxy-3,5-diiodophenoxy)-3,5-diiodophenyl] sodium propionate and the active form is the L-isomer which was originally used mainly from extracts of thyroid gland from pigs.

In recent years animal levothyroxine sodium has almost been completely replaced by synthetically prepared levothyroxine sodium, which is mainly the raw material for pharmaceutical use in treatment of diseases of the thyroid gland.

Since the thyroxin dose is small, with narrow therapeutic index, changes in the course of treatment at various times are required to adjust the dose of thyroid hormone. So, the stability of levothyroxine sodium content for a valid period of time is important for the disease treatment.

In order to manufacture solid pharmaceutical compositions, it is necessary to mix the active ingredient with inactive ingredients which may serve as binders, fillers, disintegrating agents, lubricants, or have other purposes.

Inactive ingredients are also known as "excipients". The following general criteria are essential for excipients:
- physiological inertness;
- physical and chemical stability;
- conformance to regulatory agency requirements; and
- no interference with drug bioavailability;

In reality, no single excipient would satisfy all the criteria and therefore a compromise of the different requirements has to be made.

One of the requirements for an acceptable pharmaceutical composition is that it must be stable, so as not to exhibit substantial decomposition of the active ingredient during the time between manufacture of the composition and usage by the patient.

Therefore, stability is considered one of the most important requirements of pharmaceutical product quality. Only stable preparations would promise precise delivery of the drug to the patients. Expiration dating of any drug product is based upon scientific studies at normal and stressed conditions of certain batches and/or strengths of products in case of multiple strengths. Levothyroxine is such an example because products of multiple strengths are available.

Being aware that Levothyroxine, a levo-isomer of thyroxin, is an active physiological substance secreted by the thyroid gland, there have been several attempts to prepare stable solid forms of levothyroxine sodium as an active ingredient by using different excipients and manufacturing processes.

Levothyroxine has three ionizable moieties: a carboxyl group (pKₐ = 2.4), a phenolic group (pKₐ = 6.87) and an amino group (pKₐ = 9.96), its aqueous solubility reduces from pH 1 to 3 and increases above pH 7 (Patel et al., 2003).

Previous studies have shown that different dosage forms of levothyroxine are susceptible to degradation under the influence of various environmental stress factors such as humidity, air and temperature. Won (1992) reported that levothyroxine degrades at high temperature and extreme pH.

Levothyroxine was reviewed by FDA Advisory Committee meetings where the clinical consequence/experience of products that were in the market and had approved specification limits in the range of 90-110% were reported as a problem. Moreover, there were numerous recalls of levothyroxine due to stability issues (FDA, 2006).

Furthermore, because of the lack of potency and stability assurances of the already in the market levothyroxine sodium products, the physicians doubted the switchability of these products (Thyroid, 2004).

Due to the high potency of most of the thyroid hormones, their amount when they are administered as active ingredients will generally be small, usually less than about 1% by weight. In this case, the minimum amount of thyroid hormone can vary, so long as an effective amount is provided to cause the desired therapeutic effect. Generally, the thyroid hormone will be administered to humans in an amount from about 0.012 mg to about 0.3 mg per dosage form.

Although levothyroxine sodium is a substance sensitive to light, humidity, air and temperature, it generally shows remarkable stability in the pure form.

However, in many cases its stability is dramatically reduced after mixing with various excipients necessary for the formulation in a pharmaceutical preparation, even when the preparation is provided in a solid form, i.e. tablets, capsules, powder.

Rhodes (1998) reviewed the regulatory aspects of the formulation and evaluation of levothyroxine tablets. He reported that the stability of levothyroxine tablets is a complex problem and a degradation study would reveal these stability problems resulting in the optimization of the formulation and the manufacturing process.

Various pharmaceutical preparations which contain levothyroxine sodium are already known. However, pharmaceutical preparations containing levothyroxine sodium showed a relatively short shelf life because of light, heat, air and moisture which affect the stability of the formulation.

More specifically, levothyroxine sodium hormone is hygroscopic and degrades rapidly under conditions of high humidity, in the presence of oxygen or light and under high temperature conditions.

Moreover, levothyroxine is known to degrade in the presence of certain pharmaceutical excipients such as carbohydrates, including lactose, sucrose, dextrose and starch, as well as certain dyes.

This is due to the incompatibility of levothyroxine sodium with a great number of the excipients used in the formulations, mainly due to de-iodination of the molecule and various chemical reactions which take place e.g. in the Maillard reaction.

This instability of the molecule of levothyroxine sodium and incompatibility with many common pharmaceutical excipients are responsible not only for the shorter shelf life of the Levothyroxine sodium products but also for the ineffectiveness of many of these formulations to the function of the thyroid gland, although patient receives the required (prescribed) amount of the hormone.

There have been made various efforts to solve the above issues, in particular the stability, leading to certain inventions. Gleaning some of these inventions,

WO 1999059551 A1 describes the coating of lactose with gelatin to exclude direct contact of levothyroxine sodium with lactose which is the dominant excipient. Some of the disadvantages of using gelatin are its high hygroscopicity and that gelatin undergoes a cross-linking reaction that reduces its solubility and may reduce its bioavailability.

US 5,635,209 discloses a composition comprising levothyroxine sodium and potassium iodide. Potassium iodide provides iodide ion which is included in the active substance (levothyroxine sodium). Although iodide is essential for health, supplementation with this nutrient is usually not recommended when taken with levothyroxine for a poorly functioning thyroid gland.

DE 19541128 discloses levothyroxine preparations stabilized by sodium thiosulfate. Patients may however have high sensitivity to sodium thiosulfate and sodium thiosulfate may cause allergic reactions in some patients.

US 5,225,204 describes a complex formulation of hydrated levothyroxine sodium which includes Poloxamer or polyvinylpyrrolidone and is granulated with a polar organic solvent before being uniformly adsorbed on a cellulose compound. However, no data on the stability of these formulations was provided.

US 5,955,105 describes thyroid hormone preparations stabilized by an inorganic salt, a carbohydrate with a molecular weight greater than 500, or glycine.

US 5,753,254 relates to a solid fast dispersing dosage form for oral administration which disintegrates in the mouth, wherein the dosage form comprises a thyroid hormone, a disintegrating agent, a flavoring agent and a lubricating agent.

US 6,399,101 proposes a pharmaceutical preparation of thyroid hormone and a process for tableting this preparation via direct compression. In a preferred embodiment, the pharmaceutical preparation comprises levothyroxine sodium, sodium starch glycolate, pregeletatinized starch or cellulose and silicified microcrystalline cellulose (Prosolv^{®} 50 or 90, SMCC). The SMCC is a highly porous material and functions as a carrier for levothyroxine sodium and is reported to provide improved uniformity of the pharmaceutical product. SMCC however will not provide sufficient stability if used alone.

US 6,399,101 declares that although microcrystalline cellulose (MCC) is widely used as a filler and binder for pharmaceutical formulations prepared by both wet granulation and direct compression processes, as set forth in the US. 5,955,105, patent, the combination of levothyroxine and microcrystalline cellulose as disclosed in the US. 5,225,204 patent does not provide adequate stability. Moreover, microcrystalline cellulose used in direct compression tableting has a number of limitations, such as low bulk density, high lubricant sensitivity, poor flow characteristics and the influence of moisture on the compression characteristics.

US 7,955,621 B2 describes a stable pharmaceutical formulation comprising (a) an effective amount of levothyroxine sodium, (b) microcrystalline cellulose which has a mean particle size of less than 125 µm in an amount of 60 to 85% w/w based upon the total weight of the formulation, and (c) pregelatinised starch in an amount of 5 to 30% w/w based upon total weight of the formulation.

US 8,293,272 B2 a solid pharmaceutical preparation containing water-soluble salts of levothyroxine and/or lothyronine as active ingredients wherein the water activity is adjusted to values less than 0.4 and, preferably, in the range of 0.1 to 0.3, determined at room temperature. The stability is thus provided by extracting water from the active ingredient, while it is reported that the stability is largely independent of the addition of auxiliary agents.

US 7,067,148 discloses a pharmaceutical composition in a solid dosage form comprising a thyroid hormone salt suitable for oral consumption that comprises a β-sheet form of microcrystalline cellulose as filler. It is reported that the potency loss of the composition with the β-sheet form is 3.5% versus 16.0% with the α-form microcrystalline cellulose after 15 months exposure at 25°C.

Commercial preparations currently marketed as Euthyrox, Levothroid, Levoxyl, Synthroid and Levo-T that contain different excipients (Table 1) and are manufactured via different production processes aim in stability, uniformity and dissolution characteristics which will result in efficacy and safety of the target products.

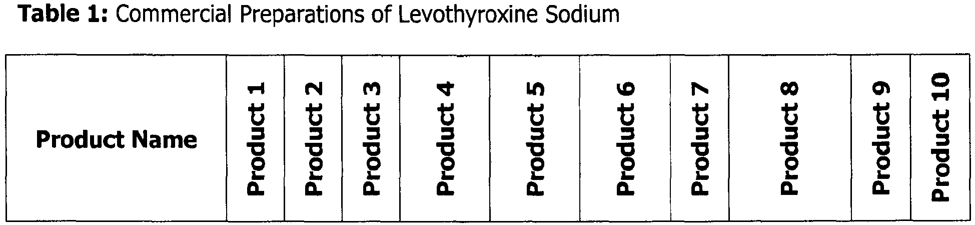

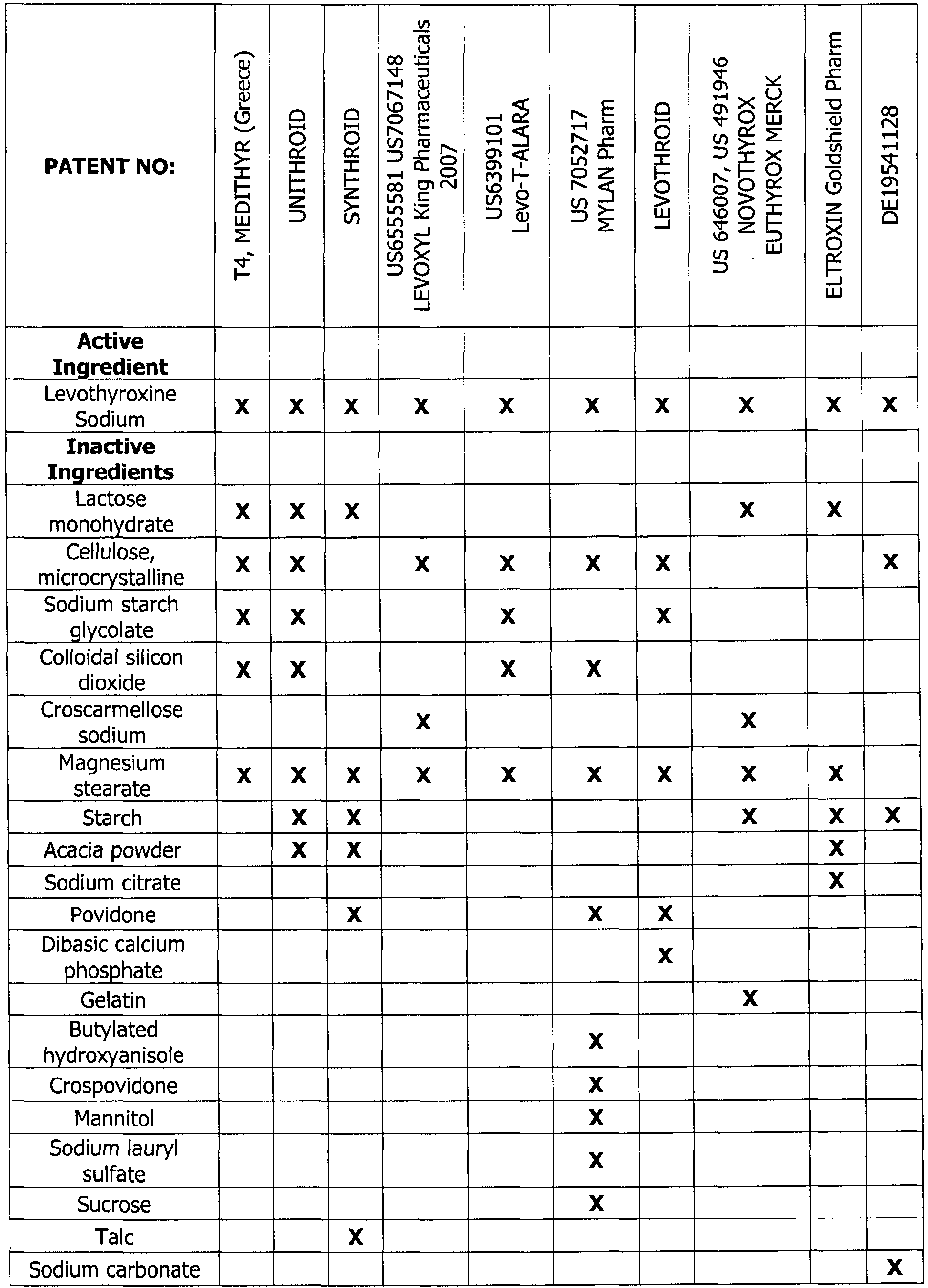

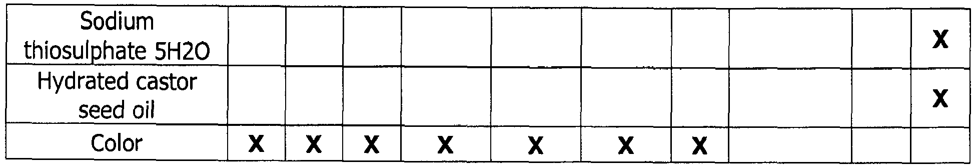

Although the known formulations may improve the stability of the preparation, pharmaceutical preparations containing levothyroxine hormone are still known to exhibit deficiencies regarding to uniformity, stability and shelf life. Thus, even though all the known attempts do provide some stability improvement, they still do not provide sufficient stability over the required storage time and further do not guarantee homogeneity, which is difficult to maintain taking into account the relatively low dosage of the thyroid hormones.

Therefore, there is still a need in the art for a stable, uniform formulation of levothyroxine which can be easily turned into dosage forms and substantially free of the disadvantages, defects and limitations of the formulations disclosed in the previews art.

The wet granulation methods using water, has more stringent requirements, as water usage leads to decreased formulation stability. The actual production via drying process is easier for moisture control.

This degradation of levothyroxine results in decreased efficiency of the drug formulation. Therefore, there is a need in the art for a levothyroxine formulation wherein levothyroxine sodium (the active ingredient) will not react with excipients resulting in the increased stability of the product.

As shown above, there have been several attempts to create stable solid forms with levothyroxine sodium as active ingredient, but either these forms are manufactured via complicated processes or the stability of the proposed forms was not comparative to the compositions of the present invention.

Summarizing, an object of the present invention is to provide a stable thyroid hormone preparation which resists degradation by light, heat, air and humidity. The stable thyroid hormone preparation is prepared with commonly used excipients and can be readily formed into a dosage formulation via a simple manufacturing process (direct compression) that improves the product performance of levothyroxine sodium tablets in terms of uniformity, stability and dissolution during acceptable shelf life.

### Summary of the Invention

The present invention provides a stable, solid oral pharmaceutical composition which is provided as an immediate release dosage form and comprises:
(i) about 0.01 % w/w to about 1% w/w levothyroxine sodium,
(ii) about 80 % w/w to about 95 % w/w α-form microcrystalline cellulose,
(iii) about 5 % w/w to about 10 % w/w disintegrant,
(iv) about 0.5 % w/w to about 3 % w/w glidant, and
(v) about 0.5 % w/w to about 2 % w/w lubricant.

The pharmaceutical composition preferably contains croscarmellose sodium as disintegrant. It further preferably contains colloidal silicon dioxide as glidant. It further preferably contains magnesium stearate as lubricant. It is further preferable that the pharmaceutical composition does not contain lactose or starch derivatives such as sodium starch glycolate that are frequently used in such compositions.

In a further preferred embodiment, the pharmaceutical composition does not contain any further excipients or additives than α-form microcrystalline cellulose, croscarmellose sodium, colloidal silicon dioxide and magnesium stearate. Thus the pharmaceutical composition may only consist of
(i) about 0.01 % w/w to about 1% w/w levothyroxine sodium,
(ii) about 80 % w/w to about 95 % w/w α-form microcrystalline cellulose,
(iii) about 5 % w/w to about 10 % w/w croscarmellose sodium,
(iv) about 0.5 % w/w to about 3 % w/w colloidal silicon dioxide, and
(v) about 0.5 % w/w to about 2 % w/w magnesium stearate.

The pharmaceutical composition may preferably contain 5 µg to 600 µg of levothyroxine sodium. In a preferred embodiment, the pharmaceutical may be prepared via dry blending and packed in a suitable container. In another preferred embodiment the composition is provided in the form of a tablet, prepared via direct compression and packed in a suitable container.

The pharmaceutical composition may be packed, as single dose, preferably in aluminum blister - PVDC, PCTFE aluminum (ACLAR) and Alu-Alu Blister material or as multi-dose, in a container suitable for multi-dose packaging.

The stable pharmaceutical composition is preferably suitable for the treatment of thyroid disorders wherein no more than 8 % of potency loss of levothyroxine sodium is determined in the pharmaceutical composition after storage for six months at 40°C and 75% relative humidity.

The pharmaceutical composition is preferably prepared with the steps of:
(i) preparation of levothyroxine sodium with a portion of the microcrystalline cellulose by geometric dilution,
(ii) mixing the result of step (i) with the remaining components of the pharmaceutical formulation,
(iii) compression of levothyroxine sodium tablets and
(iv) packing the tablets in a suitable container.

### Detailed Description of the Invention

The present invention provides a novel composition for tablets of levothyroxine sodium which exhibit extremely high stability and long shelf life.

By selecting the appropriate excipients, such as, microcrystalline cellulose, croscarmellose sodium and the appropriate manufacturing process, the stability of the levothyroxine sodium tablets can be improved, so that the stability of the formulation is substantially extended by reducing significantly the influence of heat, light and air, and humidity on the formulation and, therefore, the efficiency of the product is maintained reliably during the shelf life.

Levothyroxine sodium, as the active ingredient, participates in the compositions of this invention in an amount of 0.01% w/w - 1% w/w. In an embodiment, the compositions may contain 0.01% w/w - 0.30 % w/w, 0.01% w/w - 0.22 % w/w or 0.01% w/w - 0.11 % w/w levothyroxine sodium. The compositions may thus contain 0.01%, 0.0275%, 0.055%, 0.0825%, 0.11%, 0.22% or 1% w/w levothyroxine sodium.

The total amount of levothyroxine sodium in the formulation depends on the strength of each tablet needed to personalize the dosage for each patient, e.g. 5, 12, 25, 50, 62, 75, 88, 100, 112, 125, 137, 150, 175, 200, 300 and 600 µg levothyroxine sodium. The pharmaceutical composition may thus contain 5 µg to 600 µg of levothyroxine sodium.

In a preferred embodiment, the levothyroxine sodium will be the levothyroxine sodium pentahydrate.

It has been shown in the present invention that with the use of about 0.01 % w/w to about 1% w/w levothyroxine sodium, a high stability of the resulting pharmaceutical composition can be obtained.

The levothyroxine sodium may have a particle size in the range of 1 µm and 20 µm. Thus, levothyroxine sodium may be used with at minimum 80% of particle size in the range of 1 µm and 20 µm.

Further to the active ingredient Levothyroxine Sodium, excipients such as fillers, disintegrants, diluents, glidants, lubricants, etc. can be used in the formulation of pharmaceutical compositions.

The excipients involved in the compositions of the present invention can be classified as follows:
i) formulation excipients,
ii) excipients that facilitate formatting,
iii) excipients that improve pharmacokinetics,
iv) excipients that stabilize levothyroxine sodium.

In the present invention, the main excipient and component of the pharmaceutical levothyroxine sodium composition is the α-form of the microcrystalline cellulose. The α-form of the microcrystalline cellulose will be used in an amount of about 80% w/w to about 95% w/w.

It has herein been shown that pharmaceutical compositions comprising about 0.01 % w/w to about 1% w/w levothyroxine sodium and about 80% w/w to about 95% w/w α-form microcrystalline cellulose are particularly stable. This is particularly true if no excipients such as lactose monohydrate or sodium starch glycolate is present in the pharmaceutical composition. Two examples of the α-form microcrystalline cellulose are the type PH 101 and PH 103 that are used in the examples of the present invention. Numerous types of microcrystalline cellulose (MCC) are available on the market for a variety of uses in pharmaceutical industry. The stability of APIs will be different when in contact with different type of MCC. Some of the MCC which can be used in the production of levothyroxine sodium tablets are provided in Table below:

| | Average Particle Size by laser diffraction (µm) | Bulk Density (g/cm³) | Main Application |
|---|---|---|---|
| VIVAPUR® 101 | 65 | 0.26 - 0.31 | Fine standard MCC grade, especially suited for wet granulation, roller compaction and spheronization. Very high compactibility. |
| VIVAPUR® 301 | 65 | 0.35 - 0.46 | Same quality as grade 101, but **increased bulk density** and improved flow properties. |
| VIVAPUR® 103 | 65 | 0.26 - 0.34 | Same quality as grade 101/50M, but **very low moisture** content (< 1.5% resp. 3%) for processing water-sensitive actives. |
| VIVAPUR® 102 | 100 | 0.28 - 0.33 | Medium size standard MCC grade, suited for the majority of directly compressible actives. Combines good flow and high compactibility. |
| VIVAPUR® 105 | 25 | 0.20 - 0.26 | Very fine grade |
| **ARBOCEL®M80** | 60 | 0.20 - 0.24 | Fine, fibrous grade of powdered cellulose, suitable for wet granulation. |

It has also been shown that by deviating from the ratios and compositions with α-form microcrystalline cellulose and β-sheet microcrystalline cellulose known in the art (cf. in particular US 7,067,148), the stability of the composition can be improved. Thus, by providing the specific ratio of the active ingredient and excipients of the present invention, the stability of pharmaceutical compositions can be improved when levothyroxine sodium is used with α-form microcrystalline cellulose.

Further to the above active ingredient and α-form microcrystalline cellulose, suitable disintegrants, glidants and lubricants are used in the present compositions.

The disintegrant will be used in an amount of about 5 % w/w to about 10 % w/w, the glidant in an amount of 0.5 % w/w to about 3 % w/w and the lubricant in an amount of about 0.5 % w/w to about 2 % w/w.

Suitable disintegrants include crosslinked polyvinylpyrrolidone (crospovidone), crosslinked sodium carboxymethyl cellulose (croscarmellose sodium) and starch sodium starch glycolate. In the present invention croscarmellose sodium is particularly preferred.

Suitable glidants include colloidal silicon dioxide, talc, fumed silica and magnesium carbonate. In the present invention, colloidal silicon dioxide is particularly preferred.

Suitable lubricants include magnesium stearate, zinc stearate, sodium stearate fumarate, sodium lauryl sulfate, magnesium lauryl sulfate, talc or silica, vegetable stearin or stearic acid. In the present invention, magnesium stearate is particularly preferred.

It has herein been shown that in particular the use of sodium starch glycolate and/or lactose monohydrate may negatively affect the stability of a pharmaceutical composition. Thus it is preferred that the pharmaceutical composition does not contain any starch derivative such as sodium starch glycolate and/or lactose as excipient.

Thus in a preferred embodiment of the present invention, the stable, solid oral pharmaceutical composition which is provided as an immediate release dosage form comprises:
(i) about 0.01 % w/w to about 1% w/w levothyroxine sodium, as active ingredient, and the following pharmaceutically acceptable excipients:
(ii) about 80 % w/w to about 95 % w/w α-form microcrystalline cellulose as filler/disintegrant/diluent.
(iii) about 5 % w/w to about 10 % w/w croscarmellose sodium as disintegrant,
(iv) about 0.5 % w/w to about 3 % w/w colloidal silicon dioxide as glidant,
(v) about 0.5 % w/w to about 2 % w/w magnesium stearate as lubricant.

The pharmaceutical can be prepared via dry blending and packed in a suitable container. In another preferred embodiment the composition is provided in the form of a tablet, prepared via direct compression and packed in a suitable container.

The pharmaceutical composition may be packed, as single dose, preferably in aluminum blister - PVDC, PCTFE aluminum (ACLAR) and Alu-Alu Blister material or as multi-dose, in a container suitable for multi-dose packaging.

The stable pharmaceutical composition is preferably, suitable for the treatment of thyroid disorders wherein no more than 8 % of potency loss of levothyroxine sodium is determined in the pharmaceutical composition after storage for six months at 40°C and 75% relative humidity.

The preparation of pharmaceutical compositions with levothyroxine sodium and the determination of the levothyroxine sodium content were performed as follows:

### Quantitative Determination of the Levothyroxine sodium-Assay (According to BP Tablet Monograph)

A high performance liquid chromatography (HPLC) method is used.

### Chromatographic conditions

| | |
|---|---|
| Detector: | 225 nm |
| Column: | A stainless steel column (250 cm x 4.6 mm) packed with nitrile silica gel for chromatography (5µm), Room Temperature |
| Flow rate: | 1.0 ml/min |
| Mobile Phase: | H₂O:CH₃CN:H₃PO₄(700:300:5) |

### Preparation of pharmaceutical compositions with levothyroxine sodium

The pharmaceutical composition is preferably prepared with the steps of:
(i) preparation of levothyroxine sodium with a portion of the microcrystalline cellulose by geometric dilution,
(ii) mixing the result of step (i) with the remaining components of the pharmaceutical formulation,
(iii) compression of levothyroxine sodium tablets and
(iv) packing the tablets in a suitable container.

All the ingredients are pharmaceutical grade with a desired particle size for direct compression. The excipients are selected and their contribution to the development of compositions containing levothyroxine sodium is the following:

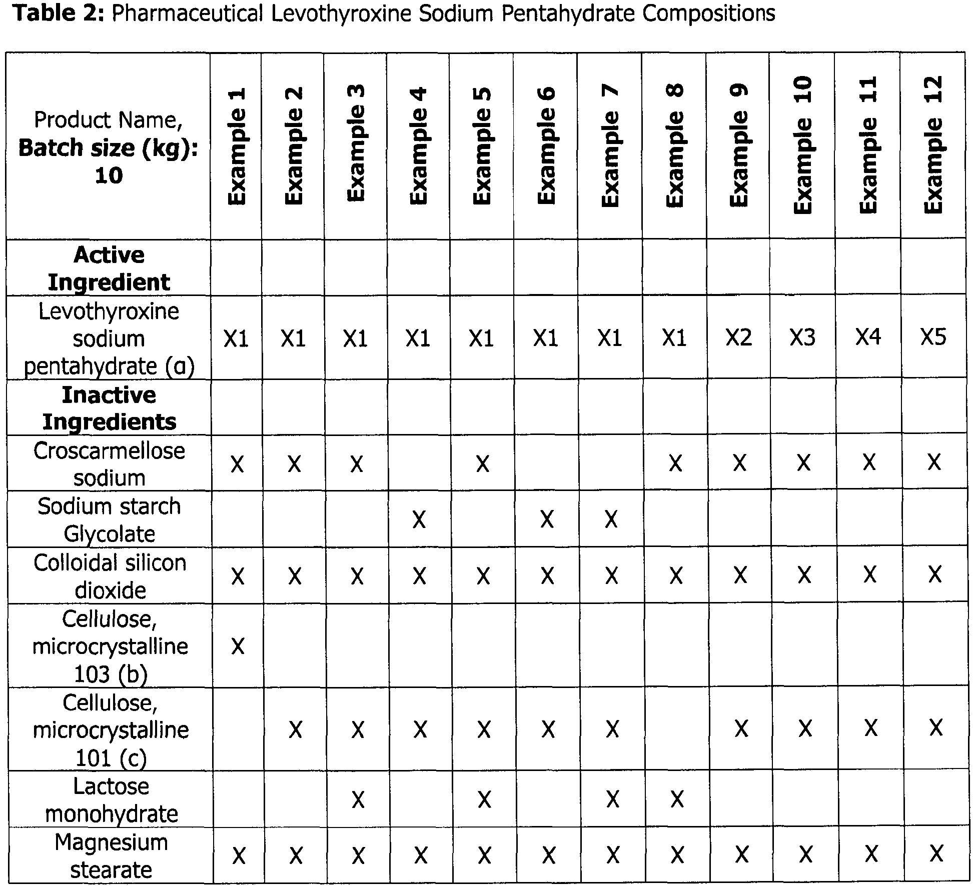

In Table 2, X1 corresponds to a content of 100 µg per tablet, X2, X3, X4 and X5 correspond to 25, 50, 75, 200 µg per tablet (cf. examples 9, 10, 11 and 12 in Table 3).

The invention will now be fully explained by the following examples. However, the scope of the invention is not intended to be limited to these examples.

### Manufacturing Process

Levothyroxine sodium tablets were manufactured via a direct compression process comprising the following steps:
I. Preparation of a levothyroxine sodium triturate with a portion of the microcrystalline cellulose by geometric dilution in the ratio provided in Table 3,
**II.** Mixing the triturate with the remaining components of the pharmaceutical formulation,
**III.** compression of levothyroxine sodium tablets at a crushing strength of not less than 3 kp and
IV. packing tablets in a suitable container (Blister, Bottles).

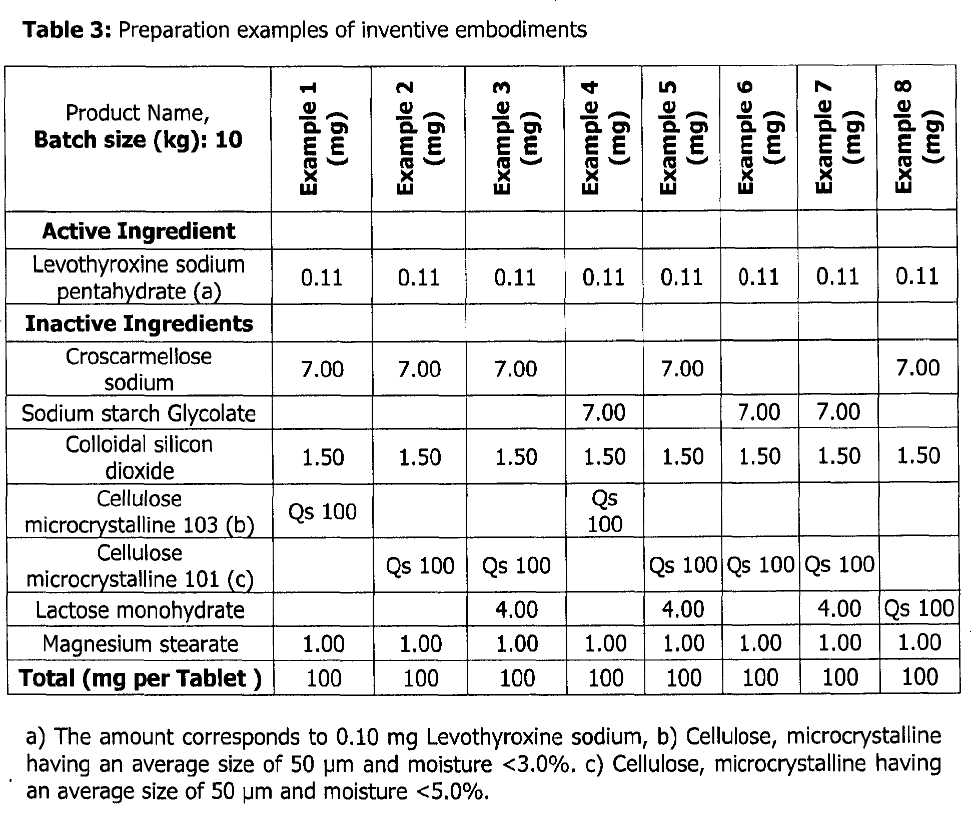

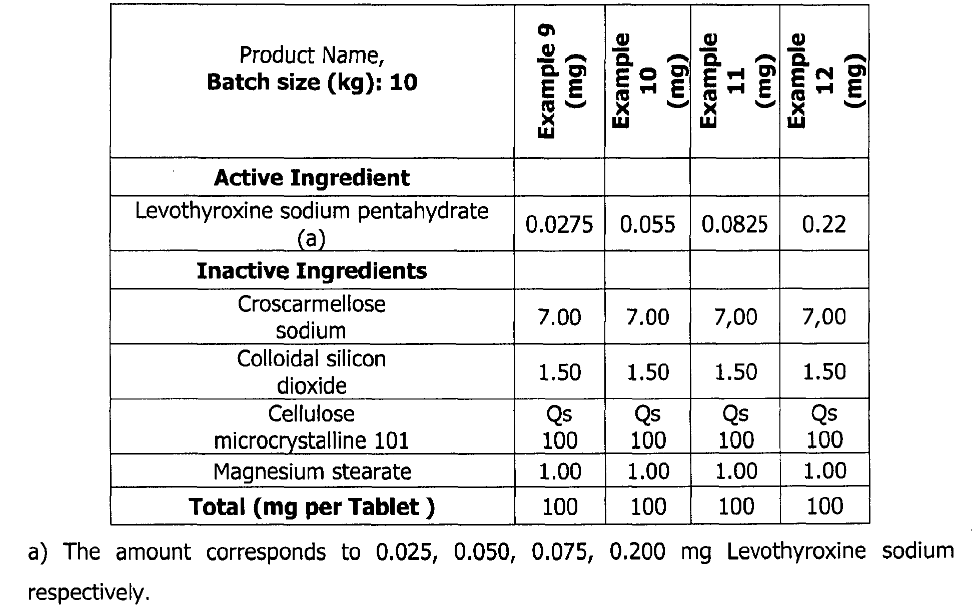

### Stability studies and results

The compositions of Examples 1-12 in Table 3 were formulated into tablets and introduced into a container suitable for single dose and multi dose. Levothyroxine sodium content of the present examples is 10-200 µg / Tab.

Primary packing materials in blister were:
1. Aluminum blister - PVDC,
2. PCTFE aluminum (ACLAR), and
3. Alu-Alu Blister.

These were placed under controlled conditions (temperature and humidity) to make the required stability studies.

Conditions:
Temperature 40°C ± 2°C, Humidity 75% ± 5%, 6-month period (Table 4-6) and
Temperature 25°C ± 2°C, Humidity 60% ± 5%, 24-month period (Table 7-9).

For this study Stability-Indicating methods for Assay, Dissolution, and Content Uniformity of Sodium Levothyroxine in Tablets were used.

The results of these stability tests are provided in Tables (4) to (9).

**Table 4: Stability results in Packaging Aluminum blister - PVDC**

| Conditions: Temperature 40°C ± 2°C, Humidity 75% ± 5%, 6-month period. Packaging : Aluminum blister - PVDC | **Assay of Levothyroxine sodium (%)** | | | | **Dissolution % release** **(Average of 12 Tablets),** **(HCl. 0,01M)** **Not less than 80% (Q)** | | |
|---|---|---|---|---|---|---|---|
| | **At the time of Batch Release** | **Uniformity of Dosage units - Content uniformity, AV<L1 % (L1=15) (Eur. Ph. 2.9.40)** | **After 6 months** | **Reduction %** | **At the time of Batch Release** | **After 6 months** | **Reduction %** |
| **Example 1** | 101,2 | Complies | 92,1 | 9,1 | 91 | 81 | 10 |
| **Example 2** | 100,6 | Complies | 91,2 | 9,4 | 90 | 81 | 9 |
| **Example 3** | 99,8 | Complies | 86,6 | 13,2 | 92 | 79 | 13 |
| **Example 4** | 99,6 | Complies | 87,2 | 12,4 | 87 | 76 | 11 |
| **Example 5** | 100,3 | Complies | 89,1 | 11,2 | 90 | 79 | 11 |
| **Example 6** | 99,4 | Complies | 85,1 | 14,3 | 86 | 69 | 17 |
| **Example 7** | 99,6 | Complies | 83,2 | 16,4 | 88 | 75 | 13 |
| **Example 8** | 98,9 | Complies | 77,1 | 21,8 | 83 | 65 | 18 |
| **Example 9** | 99,9 | Complies | 90,5 | 9,4 | 93 | 85 | 8 |
| **Example 10** | 100,2 | Complies | 91 | 9,2 | 92 | 85 | 7 |
| **Example 11** | 100,9 | Complies | 92 | 8,9 | 90 | 82 | 8 |
| **Example 12** | 99,7 | Complies | 91,9 | 7,8 | 90 | 80 | 10 |

**Table 5: Stability results in Packaging PCTFE aluminum (ACLAR)**

| Conditions: Temperature 40°C ± 2°C, Humidity 75% ± 5%, 6-month period. Packaging : PCTFE aluminum (ACLAR) | **Assay of Levothyroxine sodium (%)** | | | | **Dissolution % release** **(Average of 12 Tablets),** **(HCl. 0,01M)** **Not less than 80% (Q)** | | |
|---|---|---|---|---|---|---|---|
| | **At the time of Batch Release** | **Uniformity of Dosage units - Content uniformity, AV<L1 % (L1=15) (Eur. Ph. 2.9.40)** | **After 6 months** | **Reduction %** | **At the time of Batch Release** | **After 6 months** | **Reduction %** |
| **Example 1** | 101,2 | Complies | 94,1 | 7,1 | 91 | 83 | 8 |
| **Example 2** | 100,6 | Complies | 92,4 | 8,2 | 90 | 81 | 9 |
| **Example 3** | 99,8 | Complies | 90,6 | 9,2 | 92 | 79 | 13 |
| **Example 4** | 99,6 | Complies | 87,3 | 12,3 | 87 | 76 | 11 |
| **Example 5** | 100,3 | Complies | 92,4 | 7,9 | 90 | 82 | 8 |
| **Example 6** | 99,4 | Complies | 87 | 12,4 | 86 | 71 | 15 |
| **Example 7** | 99,6 | Complies | 85,5 | 14,1 | 88 | 75 | 13 |
| **Example 8** | 98,9 | Complies | 82,1 | 16,8 | 83 | 69 | 14 |
| **Example 9** | 99,9 | Complies | 91,2 | 8,7 | 93 | 85 | 8 |
| **Example 10** | 100,2 | Complies | 92,1 | 8,1 | 92 | 84 | 8 |
| **Example 11** | 100,9 | Complies | 93,2 | 7,7 | 90 | 81 | 9 |
| **Example 12** | 99,7 | Complies | 92,6 | 7,1 | 90 | 80 | 10 |

**Table 6: Stability results in Packaging Cold form Alu-Alu Blister PVC/Alu45/OPA25**

| Conditions: Temperature 40 °C ± 2°C, Humidity 75% ± 5%, 6-month period. Packaging : Cold form Alu-Alu Blister PVC/Alu45/OPA25 | **Assay of Levothyroxine sodium (%)** | | | | **Dissolution % release** **(Average of 12 Tablets),** **(HCl. 0,01M)** **Not less than 80% (Q)** | | |
|---|---|---|---|---|---|---|---|
| | **At the time of Batch Release** | **Uniformity of Dosage units - Content uniformity, AV<L1 % (L1=15) (Eur. Ph. 2.9.40)** | **After 6 months** | **Reduction %** | **At the time of Batch Release** | **After 6 months** | **Reduction %** |
| **Example 1** | 101,2 | Complies | 95,7 | 5,5 | 91 | 84 | 7 |
| **Example 2** | 100,6 | Complies | 96,1 | 4,5 | 90 | 83 | 7 |
| **Example 3** | 99,8 | Complies | 92,6 | 7,2 | 92 | 85 | 7 |
| **Example 4** | 99,6 | Complies | 89,2 | 10,4 | 87 | 76 | 11 |
| **Example 5** | 100,3 | Complies | 92,2 | 8,1 | 90 | 82 | 8 |
| **Example 6** | 99,4 | Complies | 88,1 | 11,3 | 86 | 73 | 13 |
| **Example 7** | 99,6 | Complies | 87,7 | 11,9 | 88 | 76 | 12 |
| **Example 8** | 98,9 | Complies | 85,8 | 13,1 | 83 | 73 | 10 |
| **Example 9** | 99,9 | Complies | 93,4 | 6,5 | 93 | 88 | 5 |
| **Example 10** | 100,2 | Complies | 94 | 6,2 | 92 | 86 | 6 |
| **Example 11** | 100,9 | Complies | 94,5 | 6,4 | 90 | 84 | 6 |
| **Example 12** | 99,7 | Complies | 94,4 | 5,3 | 90 | 83 | 7 |

**Table 7: Stability results in Packaging Aluminum blister - PVDC**

| Conditions: Temperature 25°C ± 2°C, Humidity 60% ± 5%, 24-month period. Packaging : Aluminum blister - PVDC | **Assay of Levothyroxine sodium (%)** | | | | **Dissolution % release** **(Average of 12 Tablets),** **(HCl. 0,01M)** **Not less than 80% (Q)** | | |
|---|---|---|---|---|---|---|---|
| | **At the time of Batch Release** | **Uniformity of Dosage units - Content uniformity, AV<L1 % (L1=15) (Eur. Ph. 2.9.40)** | **After 24 months** | **Reduction %** | **At the time of Batch Release** | **After 24 months** | **Reduction %** |
| **Example 1** | 101,2 | Complies | 93,4 | 7,8 | 91 | 82 | 9 |
| **Example 2** | 100,6 | Complies | 92,5 | 8,1 | 90 | 82 | 8 |
| **Example 3** | 99,8 | Complies | 88,6 | 11,2 | 92 | 81 | 11 |
| **Example 4** | 99,6 | Complies | 88,3 | 11,3 | 87 | 77 | 10 |
| **Example 5** | 100,3 | Complies | 89,8 | 10,5 | 90 | 80 | 10 |
| **Example 6** | 99,4 | Complies | 86 | 13,4 | 86 | 71 | 15 |
| **Example 7** | 99,6 | Complies | 83,9 | 15,7 | 88 | 77 | 11 |
| **Example 8** | 98,9 | Complies | 80,6 | 18,3 | 83 | 68 | 15 |
| **Example 9** | 99,9 | Complies | 91,7 | 8,2 | 93 | 85 | 8 |
| **Example 10** | 100,2 | Complies | 92,1 | 8,1 | 92 | 84 | 8 |
| **Example 11** | 100,9 | Complies | 93 | 7,9 | 90 | 83 | 7 |
| **Example 12** | 99,7 | Complies | 92,2 | 7,5 | 90 | 82 | 8 |

**Table 8: Stability results in Packaging PCTFE aluminum (ACLAR)**

| Conditions: Temperature 25°C ± 2°C, Humidity 60% ± 5%, 24-month period. Packaging : PCTFE aluminum (ACLAR) | **Assay of Levothyroxine sodium (%)** | | | | **Dissolution % release** **(Average of 12 Tablets),** **(HCl. 0,01M)** **Not less than 80% (Q)** | | |
|---|---|---|---|---|---|---|---|
| | **At the time of Batch Release** | **Uniformity of Dosage units - Content uniformity, AV<L1 % (L1=15) (Eur. Ph. 2.9.40)** | **After 24 months** | **Reduction %** | **At the time of Batch Release** | **After 24 months** | **Reduction %** |
| **Example 1** | 101,2 | Complies | 94,1 | 7,1 | 91 | 84 | 7 |
| **Example 2** | 100,6 | Complies | 92,4 | 8,2 | 90 | 82 | 8 |
| **Example 3** | 99,8 | Complies | 90,6 | 9,2 | 92 | 81 | 11 |
| **Example 4** | 99,6 | Complies | 87,3 | 12,3 | 87 | 77 | 10 |
| **Example 5** | 100,3 | Complies | 92,4 | 7,9 | 90 | 82 | 8 |
| **Example 6** | 99,4 | Complies | 87 | 12,4 | 86 | 72 | 14 |
| **Example 7** | 99,6 | Complies | 85,5 | 14,1 | 88 | 76 | 12 |
| **Example 8** | 98,9 | Complies | 82,1 | 16,8 | 83 | 69 | 14 |
| **Example 9** | 99,9 | Complies | 91,2 | 8,7 | 93 | 84 | 9 |
| **Example 10** | 100,2 | Complies | 92,1 | 8,1 | 92 | 85 | 7 |
| **Example 11** | 100,9 | Complies | 93,2 | 7,7 | 90 | 82 | 8 |
| **Example 12** | 99,7 | Complies | 92,6 | 7,1 | 90 | 82 | 8 |

**Table 9: Stability results in Packaging Cold form Alu-Alu Blister PVC/Alu45/OPA25**

| Conditions: Temperature 25 °C ± 2°C, Humidity 60% ± 5%, 24-month period. Packaging : Cold form Alu-Alu Blister PVC/Alu45/OPA25 | **Assay of Levothyroxine sodium (%)** | | | | **Dissolution % release** **(Average of 12 Tablets),** **(HCl. 0,01M)** **Not less than 80% (Q)** | | |
|---|---|---|---|---|---|---|---|
| | **At the time of Batch Release** | **Uniformity of Dosage units - Content uniformity, AV<L1 % (L1=15) (Eur. Ph. 2.9.40)** | **After 24 months** | **Reduction %** | **At the time of Batch Release** | **After 24 months** | **Reduction %** |
| **Example 1** | 101,2 | Complies | 96,1 | 5,1 | 91 | 85 | 6 |
| **Example 2** | 100,6 | Complies | 96,4 | 4,2 | 90 | 83 | 7 |
| **Example 3** | 99,8 | Complies | 92,7 | 7,1 | 92 | 85 | 7 |
| **Example 4** | 99,6 | Complies | 90,1 | 9,5 | 87 | 78 | 9 |
| **Example 5** | 100,3 | Complies | 92,8 | 7,5 | 90 | 82 | 8 |
| **Example 6** | 99,4 | Complies | 88,5 | 10,9 | 86 | 74 | 12 |
| **Example 7** | 99,6 | Complies | 88,8 | 10,8 | 88 | 76 | 12 |
| **Example 8** | 98,9 | Complies | 86,6 | 12,3 | 83 | 73 | 10 |
| **Example 9** | 99,9 | Complies | 93,7 | 6,2 | 93 | 87 | 6 |
| **Example 10** | 100,2 | Complies | 93,9 | 6,3 | 92 | 85 | 7 |
| **Example 11** | 100,9 | Complies | 94,7 | 6,2 | 90 | 84 | 6 |
| **Example 12** | 99,7 | Complies | 94,6 | 5,1 | 90 | 83 | 7 |

### Evaluation of stability results

The results show excellent initial assay, uniformity and dissolution for Example 2. For this formulation, an approximately 8% decrease in levothyroxine sodium assay was observed after six (6) months accelerated stability studies in all the primary packaging containers. As shown from the stability studies (Example 1, 2 and Examples 9-12) the use of the specific excipients combinations do not affect levothyroxine sodium, thus the stability of levothyroxine sodium is maintained during the shelf life of the tablets. The results of the 6 months stability studies under high temperature, humidity and light conditions indicate that the use of the specific excipients at the used amounts can significantly improve the stability of the levothyroxine sodium tablets.

An additional advantage of the preparations according to the invention is that this stability behavior represents an improvement over the lactose-based formula, where a 13.1-22 % decrease in levothyroxine sodium assay is observed under the same conditions and with the use of different types of excipients from these that are used in the current invention and are referred in the claim section.

A further advantage is that the dissolution profile met currents U. S. P. requirements as shown in Tables 4-5.

In case of Aluminum blister-PVDC packaging (Table 7), the loss of potency of inventive compositions (Examples 1, 2, 9-12) ranges between 7.5% and 8.2% whereas the loss of potency of the other compositions is larger than 10%. Furthermore, the relative difference of the loss of potency between inventive compositions and lactose based compositions (Examples 3, 5, 7 and 8) is in the range of 16%-64%. Similarly, in case of PCTFE aluminum (ACLAR) packaging (Table 8), the superiority of inventive compositions (Examples 1, 2, 9-12) is declared. Specifically, the potency of the innovative compositions is significantly (p<0.05) less reduced than the corresponding value of the lactose based compositions (Examples 3, 5, 7 and 8). Finally, in case of cold form Alu-Alu Blister PVC/Alh45/OPA25 packaging (Table 9), the potency of the innovative compositions is reduced only by 6% at maximum. On the other hand the potency of lactose based compositions (Examples 3, 5, 7 and 8) is reduced by 12% at maximum.

The stability studies showed that the inclusion of lactose in levothyroxine sodium compositions increases the instability of the compositions probably because of the Maillard reaction, and the packing is not always adequate. Although lactose monohydrate has a very low tendency to adsorb moisture, the fact that the levothyroxine sodium as a raw material contains significant amount of water may contribute to the reduced stability of the lactose based formulations, especially when a drying step is not included in the manufacturing process, e.g. in direct compression. Moreover, the momentarily increased temperatures due to the high compression forces during tableting may offer the sufficient activation energy for the Maillard reaction.

It should be also noted that the compositions containing sodium starch glycolate (SSG) were not as stable as the innovative compositions containing croscarmellose sodium (CCS). Both superdisintegrants are synthesized from polymers of glucose, i.e. starch (SSG) and cellulose (CCS). Although the chemical modifications in both cases are introduction of carboxymethyl sodium groups and cross-linking of the polymer backbone, there are differences in the chemistry/manufacturing process of these modifications that may affect the stability of the levothyroxine sodium compositions. In detail, the backbone of SSG and CCS consists of two polymers of α-glucose (amylose and amylopectin) and β-glucose, respectively. Furthermore, the degree of substitution of CCS is higher than that of SSG, and the mechanism of cross-linking is different. In both cases the substitution is performed using Williamson's ether synthesis to give the sodium salt of carboxymethylcellulose. However, in case of CCS the degree of substitution is such that approximately 3 anhydroglucose units out of 4 are substituted. A major difference from the chemistry/manufacturing process of SSG is that some of the carboxymethyl groups are used to cross-link the cellulose chains via dehydration. Thus the cross-links are carboxyl ester links rather than phosphate ester links as in SSG. It has been found that the combination of excipients (microcrystalline cellulose, croscarmellose sodium, colloidal silicon dioxide, magnesium stearate) and levothyroxine sodium provides a stable, uniform pharmaceutical preparation with excellent characteristics during its shelf life. This stabilizing mixture protects the levothyroxine sodium and provides excellent flow characteristics to the blend resulting in direct compression manufacturing (low cost and low humidity process) and in greater uniformity of the final product with dissolution characteristics that maintain during the shelf life ensuring its reliable bioperformance during the shelf life of the product.

### Conclusions

Stability of formulations over their shelf life is critical for having a quality product. The excipients, the manufacturing process, the storage conditions, and the packaging can either mitigate or enhance the degradation of the levothyroxine sodium affecting the potency. In the current invention it is reported that the excipients not only affect the tablet disintegration and dissolution but also stabilize the formulation. Selecting the appropriate excipients, such as, microcrystalline cellulose, croscarmellose sodium and the appropriate manufacturing process, the stability of the levothyroxine sodium tablets has been improved, so that the validity of the formulation is substantially extended by reducing significant the influence of heat, light and air, and humidity on the formulation and, therefore, the efficiency of the product is maintained reliably during the shelf life.

Summarizing, the stability of the levothyroxine sodium tablets and the current invention are strongly related.

## Claims

1. A stable, solid oral pharmaceutical composition which is provided as an immediate release dosage form and comprises:
(i) about 0.01 % w/w to about 1% w/w levothyroxine sodium,
(ii) about 80 % w/w to about 95 % w/w α-form microcrystalline cellulose,
(iii) about 5 % w/w to about 10 % w/w disintegrant,
(iv) about 0.5 % w/w to about 3 % w/w glidant, and
(v) about 0.5 % w/w to about 2 % w/w lubricant.

2. A pharmaceutical composition as claimed in claim 1, wherein the disintegrant is croscarmellose sodium.

3. A pharmaceutical composition as claimed in anyone of claims 1 to 2, wherein the glidant is colloidal silicon dioxide.

4. A pharmaceutical composition as claimed in anyone of claims 1 to 3, wherein the lubricant is magnesium stearate.

5. A pharmaceutical composition as claimed in claim 1, which does not contain lactose.

6. A pharmaceutical composition as claimed in claim 1, which does not contain starch derivatives.

7. A pharmaceutical composition as claimed in claims 1 to 4, which only consists of said components.

8. A pharmaceutical composition as claimed in anyone of claims 1 to 7, wherein 5 µg to 600 µg of levothyroxine sodium are contained.

9. A pharmaceutical composition as claimed in anyone of claims 1 to 8, prepared via dry blending and packed in a suitable container.

10. A pharmaceutical composition as claimed in anyone of claims 1 to 9, wherein the composition is provided in the form of a tablet, prepared via direct compression and packed in a suitable container.

11. The pharmaceutical composition as claimed in anyone of claims 1 to 10 packed, as single dose, preferably in aluminum blister - PVDC, PCTFE aluminum (ACLAR) and Alu-Alu Blister material.

12. The pharmaceutical composition as claimed in anyone of claims 1 to 10, packed, as multi-dose, in a container suitable for multi-dose packaging.

13. The pharmaceutical composition as claimed in anyone of claims 1 to 12 for use as a medicament, suitable for the treatment of thyroid disorders wherein no more than 8 % of potency loss of levothyroxine sodium is determined in the pharmaceutical composition after storage for six months at 40°C and 75% relative humidity.

14. A method for preparation of a pharmaceutical composition as claimed in anyone of claims 1 to 8, comprising the steps of:
(i) preparation of levothyroxine sodium with a portion of the microcrystalline cellulose by geometric dilution,
(ii) mixing the result of step (i) with the remaining components of the pharmaceutical formulation,
(iii) compression of levothyroxine sodium tablets and
(iv) packing the tablets in a suitable container.
